(19) Europäisches Patentamt
European Patent Office
Office européen des brevets

(11) Numéro de publication: **0 114 770**
**B1**

(12) # FASCICULE DE BREVET EUROPÉEN

(45) Date de publication du fascicule du brevet: **22.07.87**

(21) Numéro de dépôt: **84400101.6**

(22) Date de dépôt: **18.01.84**

(51) Int. Cl.⁴: **C 07 D 239/42,** C 07 D 237/20, C 07 D 401/04, C 07 D 403/04, C 07 D 491/10, A 61 K 31/50, A 61 K 31/495, A 61 K 31/505

(54) **Composés à noyau hétérocyclique diazoté, leur procédé de préparation et les médicaments, actifs sur le système nerveux central, qui en contiennent.**

(30) Priorité: **21.01.83 FR 8300954**

(43) Date de publication de la demande: **01.08.84 Bulletin 84/31**

(45) Mention de la délivrance du brevet: **22.07.87 Bulletin 87/30**

(84) Etats contractants désignés: **AT BE CH DE FR GB IT LI LU NL SE**

(56) Documents cité: **US-A-4 302 455**

(73) Titulaire: **SANOFI, société anonyme, 40, Avenue George V, F-75008 Paris (FR)**

(72) Inventeur: **Biziere, Kathleen, 6, Lotissement Rayons d'Oc, F-34170 Clapiers (FR)**
Inventeur: **Chambon, Jean- Pierre, Chemin des Truquets Route de Montpellier, F-34570 Montarnaud (FR)**
Inventeur: **Hallot, André, 83, rue du Juge, F-34980 St Gely Du Fesc (FR)**

(74) Mandataire: **Gillard, Marie- Louise, Cabinet Beau de Loménie 55, Rue d'Amsterdam, F-75008 Paris (FR)**

**EP 0 114 770 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

## Description

L'invention a pour objet des composés hétérocycliques nouveaux présentant d'intéressantes propriétés thérapeutiques sur le système nerveux central.

Les composés selon l'invention sont décrits par la formule générale:

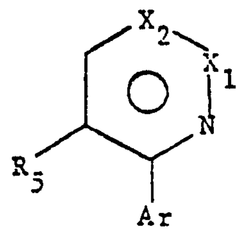

dans laquelle:

- $X_1$ représente N ou le groupe 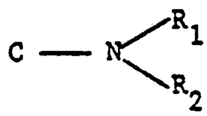 ;

- $X_2$ représente N ou le groupe 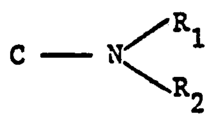

à la condition que

$X_1$ et $X_2$ soient différents l'un de l'autre

- $R_1$ et $R_2$ représentent $-(CH_2)_nOH$ ; n = 1 à 4

ou

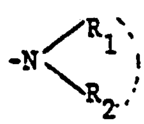

constitue un hétérocycle monoazoté saturé à 5 ou 6 chaînons et portant un substituant en position 3 ou 4 choisi parmi :

i) un groupe $-(CH_2)_mOR_6$ où m est égal à 0, 1 ou 2 et

$R_6$ représente l'hydrogène ou un groupe 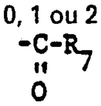

ou 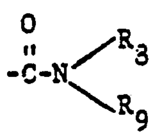

FIG6/20

dans lesquels $R_7$ représente un groupe alkyle droit ou ramifié comportant de 1 à 5 atomes de carbone, un groupe cyclopropyle ou un groupe phényle éventuellement substitué par un atome d'halogène, $R_8$ et $R_9$, considérés indépendamment, représentent chacun l'hydrogéne ou un groupe alkyle droit ou ramifié comportant de 1 à 5 atomes de carbone;

ii) un groupe oxo (=0);

iii) un groupe spiro dioxolane-1,3 yl-2 ( 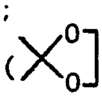 ) ;

- Ar représente:

. un groupe 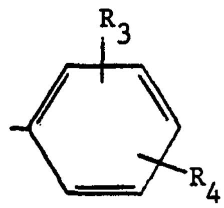 dans lequel

$R_3$ désigne l'hydrogène, un atome d'halogène, un groupe alkyle droit ou ramifié comportant de 1 à 5 atomes de carbone, un groupe alcoxy droit ou ramifié comportant de 1 à 5 atomes de carbone, un groupe trifluorométhyle, un groupe hydroxy, un groupe nitro ou un groupe cyano et

$R_4$ désigne l'hydrogène ou un atome d'halogène;

un groupe naphtyle non substitué ou monosubstitué par un atome d'halogène;

$R_5$ désigne l'hydrogène ou un atome de chlore, étant entendu que $R_5$ ne peut être un atome de chlore que si $X_2$ représente un atome d'azote;

Plus précisément, les composés selon l'invention sont représentés par l'une des deux formules suivantes:

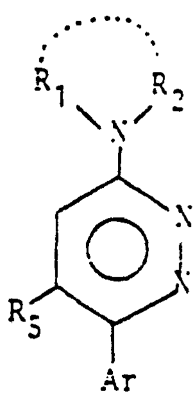

Ia

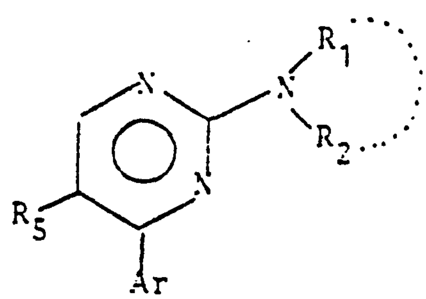

Ib

Les composés I donnent des sels avec les acides organiques ou minéraux. Ces sels avec les acides pharmaceutiquement acceptables font partie intégrante de l'invention.

Les composés 1 sont préparés à partir du dérivé cétonique correspondant obtenu selon les méthodes décrites dans la littérature. Le dérivé cétonique est transformé dans le composé aromatique chloré correspondant; puis celui-ci est transformé en composé aminé correspondant.

**Préparation de I a:**

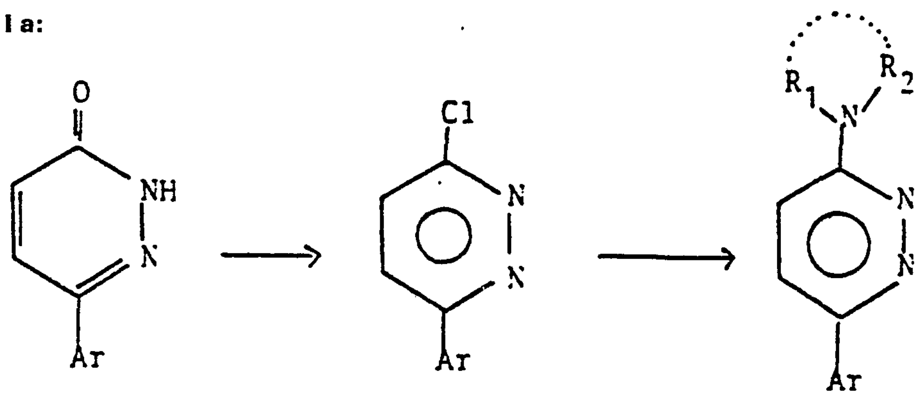

Ia

Les pyridazinones-3 sont préparées selon les méthodes décrites dans : Journal of the American Chemical Society, 1953, p 1117-1119 et Journal of Organic Chemistry, 1973, 38, p 4044-4048.

Les aryl-6 chloro-3 pyridazines sont des composés connus ils peuvent notamment être préparés selon la méthode décrite dans: Journal of Heterocyclic Chemistry, 1973, 38, 881-892.

Lorsque l'un des substituants $R_3$ ou $R_4$ est hydroxyle celui-ci doit être bloqué, par exemple par le chloroformiate d'éthyle, pendant la préparation de l'aryl-6 chloro-3 pyridazine.

La substitution de ls pyridazine par le groupe amine hydroxylée

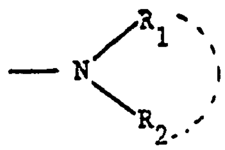

se fait par chauffage de l'aryl-6 chloro-3 pyridazine et de l'amine $HNR_1R_2$ dans un alcanol à température d'ébullition.

On peut ensuite éventuellement estérifier le produit par action d'un chlorure d'acide en présence d'une amine tertiaire en chauffant le mélange dans un solvant aprotique par exemple le tétrahydrofuranne.

On peut également préparer éventuellement le carbamate N-monosubstitué par addition sur l'amine hydroxylée d'un isocyanate et chauffage dans un solvant aprotique, par exemple le tétrahydrofuranne.

De même les carbamates N, N-disubstitués s'obtiennent par action d'un chlorure de carbamoyle au sein d'un solvant comme le dioxane à reflux et en présence d'un accepteur d'hydracide, tel que la triéthylamine.

Enfin, lorsque l'amine cyclique est substituée par un groupe oxo, le composé I s'obtient par hydrolyse acide de l'acétal cyclique correspondant.

**EXEMPLE 1**:

**(Hydroxy-4 pipéridino)-3 (nitro-2 phényl)-6 pyridazine ; SR 41673.**

En utilisant les méthodes décrites dans la littérature, on prépare la chloro-3 (nitro-2 phényl)-6 pyridazine (Fk : 138-140° C). 3 g de ce produit sont mélangés à 3,86 g d'hydroxy-4 pipéridine dans 60 ml de butanol normal et chauffés sous agitation à 100° C pendant 5 heures.

Le mélange réactionnel est concentré sous vide à siccité puis le résidu est repris à l'eau et extrait à l'acétate d'éthyle. La phase organique est lavée à l'eau, séchée sur sulfate de sodium puis concentrée à siccité. L'huile résiduelle est chromatographiée sur gel de silice en utilisant un mélange chloroforme/méthanol (90/10 en volume) comme éluant. Après évaporation des solvants, l'huile résiduelle cristallise. On recristallise dans l'acétonitrile pour obtenir 2,6 g du produit attendu. point de fusion (Fk) 138-139° C.

**EXEMPLE 2**:

**Hydroxy-4 pipéridino)-3 (chloro-2 phényl)-6 pyridazine. CM 40907.**

On opère selon les méthodes citées pour préparer la chloro-3 (chloro-2 phényl)-6 pyridazine, (Fk : 146-147° C). 5 g de ce produit sont dissous dans 120 ml de butanol normal et mélangés à 6,74 g d'hydroxy-4 piperidine puis chauffés à reflux. En suivant le mode opératoire décrit pour l'exemple précédent on obtient après recristallisation dans l'éthanol absolu 5 g de CM 40907. Fk : 154-155° C.

**EXEMPLE 3**:

**(Butyroyloxy-4 pipéridino)-3 (chloro-2 phényl)-6 pyridazine; SR 41172**.

2, 9 g de CM 40907 sont dissous dans 100 ml de tétrahydrofuranne, on ajoute 3,5 ml de triéthylamine et 2,6 g de chlorure de butyryle puis on chauffe à reflux pendant 3 jours. On rajoute 0, 7 ml de triéthylamine et 0,52 ml de chlorure de butyryle et l'on chauffe à nouveau à reflux pendant 24 heures. Le milieu réactionnel est ensuite concentré à siccité et le résidu est repris par l'acide chlorhydrique normal et lavé à l'éther.

Après addition de carbonate de sodium on extrait à l'acétate d'éthyle. La phase organique est séchée sur sulfate de sodium et concentrée à siccité. L'huile résiduelle est chromatographiée sur gel de silice en utilisant l'acétate d'éthyle comme éluant. Après concentration et recristallisation dans l'éther isopropylique on obtient 2,7 g de SR 41172. Fk : 89-90° C.

**EXEMPLE 4**:

**(Méthylcarbamoyloxy-4 piperidino)-3 (chloro-2 phéryl)-6 pyridazine; SR 41820**.

On prépare une solution de 2,7 g de CM 40907 dans 50 ml de tétrahydrofuranne. On chauffe à reflux pendant 48 heures en présence de 1,65 ml d'isocyanate de méthyle, après avoir rajouté 1,65 ml d'isocyanate de méthyle, le chauffage est repris pendant 48 heures.

Le milieu réactionnel est concentré sous vide le résidu est chromatographié sur gel de silice en utilisant l'acétate d'éthyle comme éluant. Après concentration, le produit est recristalligé dans l'acétate d'éthyle. On obtient 1 g de SR 41820. Fk: 134-136° C.

**EXEMPLE 5**:

**(Diméthylcarbamoyl oxy-4 pipéridino)-4 (dichloro-2,4 phényl)-6 pyridazine; SR 42432**.

a) - (Hydroxy-4 pipéridino)-3 (dichloro-2, 4 phényl)-6 pyridazine; SR 41378.

On opère comme dans l'exemple 1 à partir de chloro-3 (dichloro-2,4 phényl)-6 pyridazine et l'hydroxy-4 pipéridine.

De la même façon, on obtient le produit attendu F: 151-3° C (éthanol).

b) - SR 42432.

On dissout 4,7 g du produit obtenu ci-dessus dans 60 ml de dioxane puis on ajoute 6,3 ml de triétylamine et 4,1 ml de chlorure de diméthylcarbamoyle. On chauffe à reflux pendant 7 jours puis on évapore le solvant à siccité sous vide. On reprend le résidu dans une solution aqueuse de carbonate de sodium à 10 % et extrait avec de l'acétate d'éthyle. On sépare la couche organique, sèche sur sulfate de sodium et évapore le solvant à siccité sous vide. On chromatographie le résidu sur gel de silice en éluant avec de l'acétate d'éthyle. On obtient un produit qui cristallise. Après recristallisation dans l'acétate d'éthyle, F: 196-198° C ; Poids: 2,8 g

**EXEMPLE 6**:

**[Dioxa-1, 4 aza-8 spiro dec (4, 5) yl-8]-3 (chloro-2 phényl)-6 pyridazine; SR 42487**.

On chauffe au reflux pendant 18 heures le mélange de 4,5 g de chloro-3 (chloro-2 phényl)-6 pyridazine et 8,2 g de dioxa-1,4 aza-8 spiro-décane [4,5] dans 100 ml de butanol.

On évapore le butanol à siccité sous vide et on reprend le résidu dans l'eau. On extrait avec de l'acétate d'éthyle et sèche la solution organique sur sulfate de sodium. On évapore le solvant à siccité sous vide et recristallise le résidu dans l'acétate d'éthyle. F: 156-158° C; Poids: 5,2 g.

**EXEMPLE 7**:

**(Oxo-4 pipéridino)-3 (chloro-2 phényl)-6 pyridazine; SR 42488**.

On chauffe au reflux, pendant 4 heures, 4 g du produit de l'exemple 6 dans le mélange de 40 ml d'acide formique et 60 ml d'eau. On verse le mélange réactionnel sur une solution de soude en excès additionnée de glace. On extrait avec du chlorure de méthylène sépare la couche organique et sèche sur sulfate de sodium.

On évapore le solvant à siccité sous vide puis recristallise le résidu dans l'éthanol absolu. F: 158-160° C; Poids: 3,2 g.

En utilisant des méthodes analogues, on prépare les composés selon l'invention décrits dans le tableau I ci-dessous.

TABLEAU I

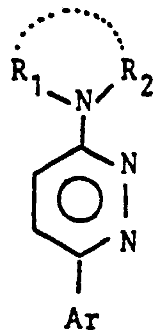

| Numéro du Produit | 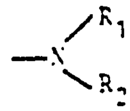 | Ar | Fk °C (solvant de cristallisation) |
|---|---|---|---|
| CM 40857 | 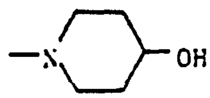 | 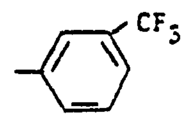 | 130°C (acétate d'éthyle) |
| CM 41096 | 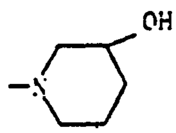 | 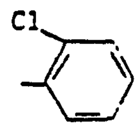 | 134°C (acétate d'éthyle) |
| CM 41127 | 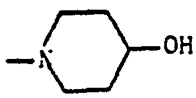 | 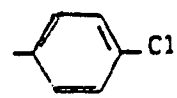 | 174-176°C (éthanol absolu) |
| SR 41155 | 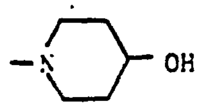 | 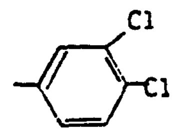 | 150-152°C (acétate d'éthyle) |
| SR 41171 | 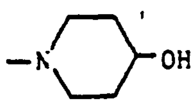 | 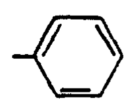 | 150°C (acétonitrile) |
| SR 41173 | 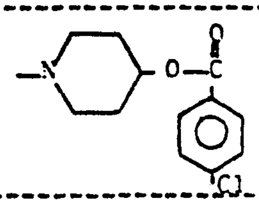 | 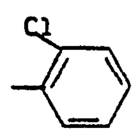 | 142 - 144°C (acétonitrile) |
| SR 41174 | 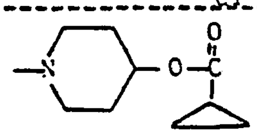 | " " | 88-90°C (chlorure de méthylène-éther isopropylique) |
| SR 41175 | 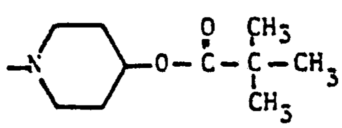 | " " | 145°C (acétonitrile) |

| | | | |
|---|---|---|---|
| SR 41184 | 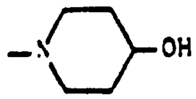 | 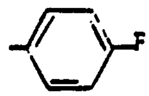 | 162-164°C (alcool éthylique) |
| SR 41185 | 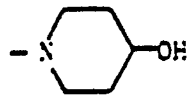 | 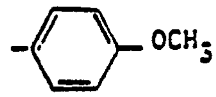 | 182-184°C (alcool éthylique) |
| SR 41188 | 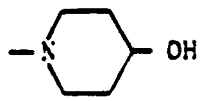 | 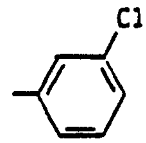 | 152-154°C (acétate d'éthyle) |
| SR 41254 | 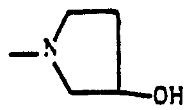 | 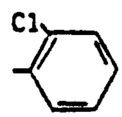 | 170-172°C (alcool éthylique) |
| SR 41259 | 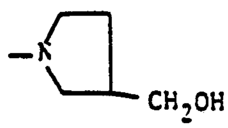 | " " | 136-138°C (alcool éthylique-éther isopropylique) |
| SR 41261 | 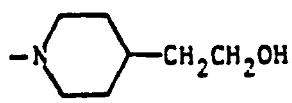 | " " | 88-90°C (isopropanol-éther isopropylique) |
| SR 41558 | 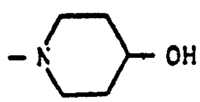 | 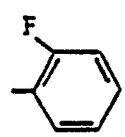 | 132-134°C (éthanol-éther isopropylique) |
| SR 41559 | 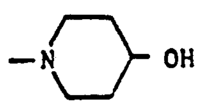 | 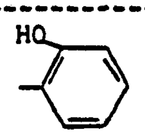 | 141-143°C (acétonitrile) |
| SR 41673 | 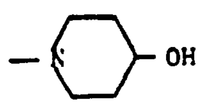 | 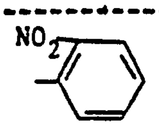 | 138-139°C (acétonitrile) |

| | | | |
|---|---|---|---|
| SR 41900 | 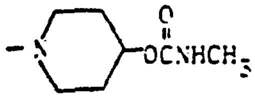 | 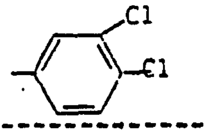 | 168-170°C (acétonitrile) |
| SR 41915 | 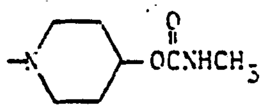 | 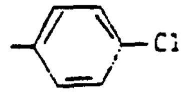 | 216-218°C (acétonitrile) |
| SR 41917 | 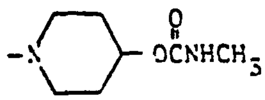 | 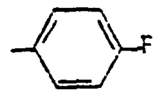 | 170-172°C (acétonitrile) |
| SR 41930 | 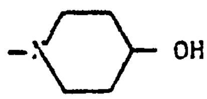 | 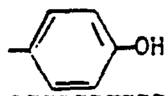 | 230°C (éthanol) |
| SR 41931 | 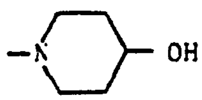 | 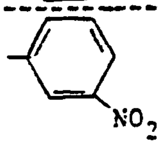 | 165°C (acétonitrile) |
| SR 41935 | 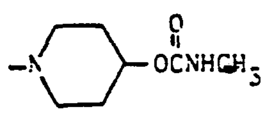 | 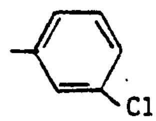 | 164-166 (acétate d'éthyle) |
| SR 41965 | 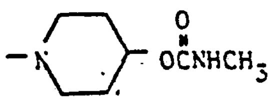 | 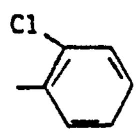 | 166-167°C (acétate d'éthyle) |
| SR 41932 | 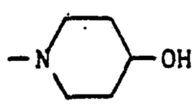 | 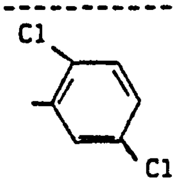 | 172-173°C (éthanol) |
| SR 42087 | 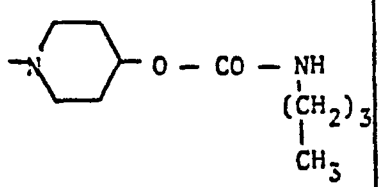 | 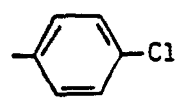 | 156-158°C (acétate d'éthyle) |

| | | | |
|---|---|---|---|
| SR 42093 | 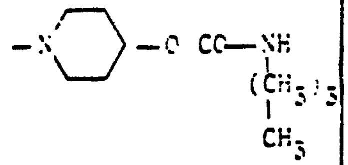 | 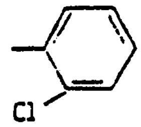 | 154-155°C (acétate d'éthyle - éther isopropylique) |
| SR 42095 | 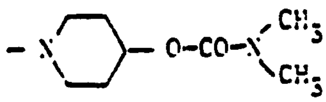 | " " | 131°C (acétate d'éthyle - éther isopropylique) |
| SR 42159 | 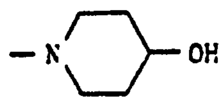 | 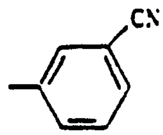 | 136-138°C (acétate d'éthyle - éther isopropylique) |
| SR 42356 | 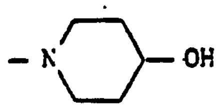 | 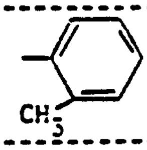 | 187-188°C (méthanol) |
| SR 42368 | 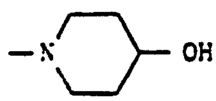 | 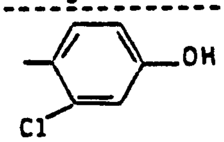 | 176-178°C (acétate d'éthyle) |
| SR 42399 | 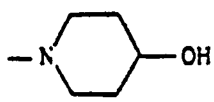 | 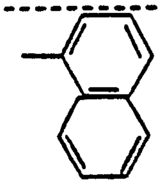 | 148-150°C (acétate d'éthyle |

**Préparation de I b: Schéma réactionnel**

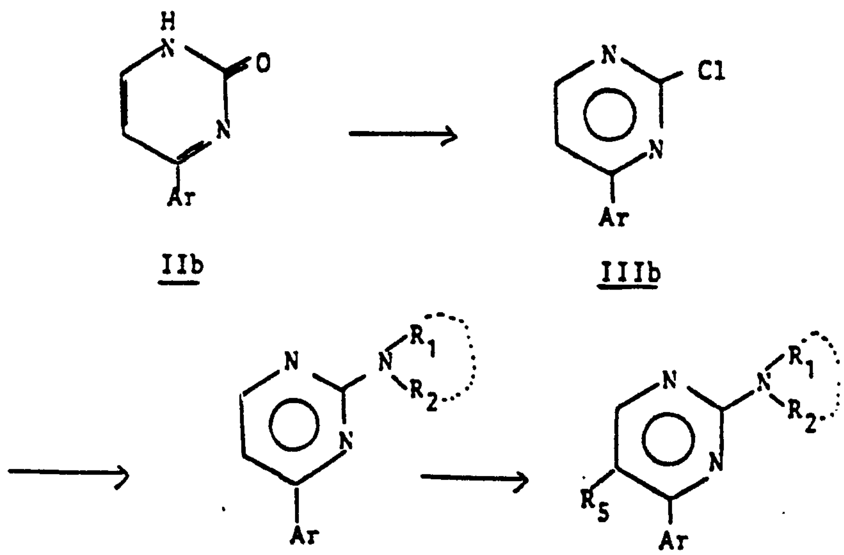

IIb IIIb IVb

Les aryl-6 pyrimidinones-2 (II b) sont préparées selon la méthode décrite dans: Journal of Organic Chemistry, 1953, 18 p 588-593.

Les composés III b et IV b sont obtenus en utilisant les mèmes méthodes que celles utilisées pour les composés semblables en série pyridazine.

Si $R_5$ est un atome de chlore, l'halogénation du noyau pyrimidine pour obtenir Ib est effectuée par le N-chloro succinimide selon la méthode décrite dans: Tetrahedron, 1966. 22, 2401.

**EXEMPLE 8:**

**(Hydroxy-4 pipéridino)-2 (chloro-2 phényl)-4 pyrimidine; CM 40724.**

a) (Chloro-2 phényl)-4 pyrimidinone-2.

La chloro-2 bénzoyl acétaldéhyde est préparée selon la méthode décrite dans : Berichte der Deutschen Chemischen Gesellschaft, 1901, 34, 3889-3897.

53,6 g de ce produit sont dissous dans 2,5 l d'alcool absolu auquel on ajoute 17,63 g d'urée et 29,35 ml d'acide chlorhydrique concentré. Après chauffage à reflux pendant une nuit le mélange réactionnel est concentré. Le résidu est repris par la soude diluée et extrait à l'acétate d'éthyle. La phase organique est lavée à l'eau, séchée sur sulfate de sodium et concentrée à siccité.

Le résidu cristallisé est chromatographié puis 10,1 g sont dissous dans 500 ml d'éthanol. On ajoute 4,54 g de potasse dissous dans 50 ml d'eau puis on chauffe à reflux 30 minutes. Après avoir concentré sous vide le mélange réactionnel, le résidu est neutralisé par l'acide chlorhydrique dilué.

La phase organique est extraite au chlorure de méthylène lavée à l'eau, séchée sur sulfate de sodium puis concentrée. Le résidu cristallisé est lavé avec 50 ml d'acétate d'éthyle pour donner 6 g de produit blanc.

Fk : 210-212° C.

b) Chloro-2 (chloro-2 phényl)-4 pyrimidine.

Ce produit est obtenu par action de l'oxychlorure de phosphore à chaud selon la méthode citée pour la préparation de I a

Fk : 100° C (hexane).

c) CM 40724.

On utilise le mode opératoire décrit dans l'exemple 1 pour ajouter l'hydroxy-4 pipéridine.

Fk: 102-104° C (éther isopropylique).

**EXEMPLE 9:**

**Bromhydrate de chloro-5 (chloro-2 phényl)-6 (hydroxy-4 pipéridino)-2 pyrimidine; SR 40858.**

2,7 g de CM 40724 sont dissous dans 50 ml de chloroforme. On ajoute 1,37 g de N-chlorosuccinimide et le mélange réactionnel est chauffé à reflux pendant une nuit puis concentré sous vide. Le résidu est repris par 50 ml d'eau bouillante le mélange légèrement refroidi est extrait par l'acétate d'éthyle. La phase organique est décantée séchée sur sulfate de sodium puis concentrée. L'huile résiduelle est chromatographiée sur gel de silice en utilisant comme éluant un mélange cyclohexane-acétate d'éthyle 50/50 en volume. Après élimination d'impuretés en tête de colonne, le produit décrit est élué. La concentration des fractions donne 1,9 g d'huile que l'on reprend dans 100 ml d'éther.

Après barbotage de gaz bromhydrique jusqu'à pH acide le bromhydrate du produit cristallise. On le recristallise dans l'acétonitrile.

Fk: 192-194° C.

En suivant les méthodes de préparation indiquées on obtient les composés selon l'invention décrits dans le tableau II ci-dessous.

**TABLEAU II**

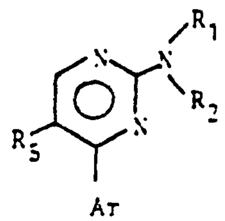

| Numéro du Produit | 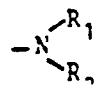 | $R_5$ | Ar | Fk°C (solvant de cristallisation |
|---|---|---|---|---|
| SR 41554 | 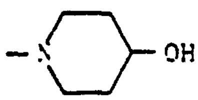 | H | 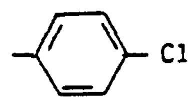 | 140-142°C (éther isopropylique) |
| SR 41556 | 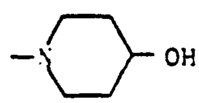 | Cl | 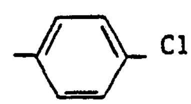 | 144°C (éther isopropylique) |
| SR 41590 | 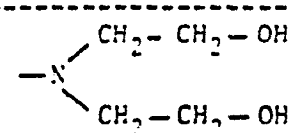 | H | 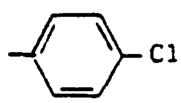 | 114°C (éther isopropylique) |
| SR 41591 | 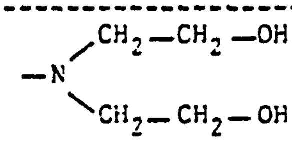 | H | 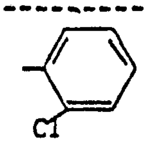 | 106-108°C (éther isopropylique) |

Les produits selon l'invention ont été évalués pour leurs effets hypnogène et sédatif, ainsi que leur activité anticonvulsivante.

## I -EVALUATION DE L'EFFET HYPNOGENE ET SEDATIF DES PRODUITS

a. <u>Effet de produits sur la motilité spontanée</u>.

L'effet sédatif se traduit par la diminution de la motilité spontanée des animaux. Celle-ci a été mesurée grâce au test d'actimétrie développée par BOISSIER et SIMON (1965). L'équipement était composé de cages actimétriques, type Apelab (26 x 21,5 x 10 cm), traversées par deux rayons lumineux qui impressionnent une cellule photo-électrique. Les lots étaient contitués de 12 souris femelles Charles River CD1, de poids compris entre 20 et 24 g. Les animaux ont été placés individuellement dans les cages 45 minutes après l'administration orale des produits à la dose de 250 mg/kg ou à la dose de 100 mg/kg. Chaque traversée d'un faisceau lumineux a été comptabilisée par un compteur individuel. Les scores correspondant aux déplacements des animaux ont été enregistrés pendant 10 minutes et comparés aux scores réalisés par les lots d'animaux témoins, uniquement traités par le véhicule (HCl 0,1 N).

<u>Commentaire</u>: Après administration, à la dose orale de 100 mg/kg ou de 250 mg/kg, les produits de l'invention révèlent un puissant effet sédatif caractérisé par une réduction importante de la motilité des animaux.

D'autre part, à la dose de 100 mg/kg, le CM 41378 provoque une perte du réflexe de retournement caractéristique d'un effet inducteur de sommeil chez 60 % des animaux traités.

**TABLEAU III**

| Produits | Dose p.o. mg/kg | Activité locomotrice Score (p.cent/témoins) |
|---|---|---|
| CM 40857 | 100 | - 87 % ** |
| CM 40907 | 100 | - 66 % ** |
| CM 41127 | 100 | - 68 % ** |
| SR 41155 | 100 | - 82 % ** |
| SR 41171 | 100 | - 34 % |
| SR 41172 | 100 | - 32 % ** |
| SR 41184 | 100 | - 91 % ** |
| SR 41188 | 100 | - 77 % ** |
| SR 41254 | 100 | - 34 % * |
| SR 41259 | 100 | - 19 % |
| SR 41261 | 100 | - 27 % * |
| SR 40858 | 250 | - 20 % |
| SR 41185 | 250 | - 78 % ** |
| SR 41554 | 250 | - 26 % * |
| SR 41556 | 250 | - 29 % ** |
| SR 41558 | 250 | - 93 % ** |
| SR 41559 | 250 | - 66 % ** |
| SR 41654 | 250 | - 57 % ** |
| SR 41673 | 250 | - 61 % ** |
| SR 41174 | 100 | - 15 % |
| SR 41932 | 150 | - 59 % ** |
| SR 42095 | 10 | - 28 % * |
| SR 42487 | 100 | - 42 % ** |

* p 0,05 (test de Student) ** p 0,01 (test de Student)

b. Potentialisation de la narcose au pentobarbital.

Dans le but d'apprécier le pouvoir hypnogène des produits nous avons étudié leur capacité à potentialiser les effets d'une dose subnarcotique de pentobarbital chez la souris. Les lots étaient constitués de 10 souris Charles River CD1 de poids compris entre 20 et 24 g. Le pentobarbital (20 mg.kg, i.p.) a été administré 60 minutes après l'administration des produits. Le critère d'endormissement retenu a été la perte du réflexe de retournement. Les animaux qui n'ont pas présenté ce réflexe ont été dénombrés.

| Produits | Potentialisation du pentobarbital $DE_{50}$ (mg/kg, p.o.) |
|---|---|
| CM 40907 | 96 |
| SR 41155 | 20 |
| SR 41378 | 21 |
| SR 41554 | 66 |
| SR 42095 | 7,4 |
| SR 42488 | 67 |

Les produits selon l'invention sont aptes à potentialiser la narcose au pentobarbital ; cette propriété est prédictive d'un effet hypnogène.

## II - EVALUATION DE L'ACTIVITE ANTICONVULSIVANTE DES PRODUITS.

L'effet anticonvulsivant des produits chez la souris a été évalué sur un modèle de convulsions provoquées par un choc électrique, et sur un modèle de convulsions induites par un agent chimique; la bicuculline.

a. Antagonisme des convulsions induites par un choc électrique.

Le test a été légèrement modifié de celui de SWINYARD et al. (1952) et ASAMI et al. (1974). Le matériel était composé d'un générateur de choce Racia muni de 2 électrodes oculaires délivrant un courant de 12,5 volts pendant 0,3 seconde. Les lots étaient constitués de 10 souris Charles River CD1, de poids compris entre 20 et 24 g. Les produits ont été administrés par voie orale 60 minutes avant l'électrochoc. Les animaux ne présentant pas l'extension tonique des membres postérieurs ont été considérés comme protégés de la crise convulsive.

b. Antagonisme des convulsions provoquées par la bicuculline.

Les lots étaient constitués de 10 souris Charles River CD1 de poids compris entre 20 et 22 g. Les produits ont été administrés par voie orale, 60 minutes avant la bicuculline (0,8 mg/kg, i.v.). L'apparition de convulsions toniques a été notée pendant les 60 minutes qui ont suivi l'injection de la bicuculline.

Commentaire:

Après administration orale chez la souris, les produits selon l'invention manifestent des propriétés anticonvulsivantes tant vis-à-vis du choc électrique que de la bicuculline.

**TABLEAU IV**

| Produits | Dose efficace médiane ($DE_{50}$) (mg/kg, p.o.) | |
|---|---|---|
| | Antagonisme du choc électrique | Antagonisme de la Bicuculline |
| CM 40857 | 63 | |
| SR 40858 | 49 | |
| CM 40907 | 16 | 38 |
| CM 41127 | 28 | 98 |
| SR 41171 | 86 | 83 |
| SR 41172 | 28 | 39 |
| SR 41184 | 48 | |
| SR 41188 | 45 | |
| SR 41378 | 30 | 5,7 |
| SR 41554 | 145 | 147 |
| SR 41558 | 115 | 30 |
| SR 41673 | 27 | 81 |
| SR 41820 | 10 | 13 |
| SR 41174 | 92 | |
| SR 41932 | 30 | 131 |
| SR 42095 | 2 | 1,6 |
| SR 42399 | 38 | |
| SR 42487 | 57 | |
| SR 42488 | 40 | 35 |

**III - DETERMINATION DE LA DOSE LETALE CHES LA SOURIS APRES ADMINISTRATION AIGUE DES PRODUITS.**

Les produits ont été administrés par voie orale, à des lots de 5 souris femelles Charles Rivers CD1, de poids compris entre 20 et 24 g. Ils ont été solubilisés dans HCl 0,1 N.

La toxicité a été relevée pendant les 72 heures qui ont suivi l'administration des produits. La dose létale 50 ($DL_{50}$) a été calculée pour deux produits.

**TABLEAU V**

| Produits | p. cent de toxicité ou $DL_{50}$ | | |
|---|---|---|---|
| | 250 mg/kg p.o. | 500 mg/kg p.o. | 1000 mg/kg p.o. |
| SR 40858 | 0 | 0 | 0 |
| CM 40907 | | $DL_{50}$ = 807 | |
| CM 41127 | 0 | 0 | |
| SR 41155 | 0 | 100 | 100 |
| SR 41171 | 0 | 0 | 100 |
| SR 41172 | 0 | 0 | 100 |
| SR 41184 | 0 | 60 | 100 |
| SR 41185 | 0 | 0 | 0 |
| SR 41188 | 0 | 0 | 60 |
| SR 41254 | 0 | 0 | 20 |
| SR 41378 | | $DL_{50}$ = 423 | |
| SR 41554 | 0 | 0 | 0 |

**TABLEAU V (suite)**

| Produits | p. cent de toxicité ou $DL_{50}$ | | |
|---|---|---|---|
| | 250 mg/kg p.o | 500 mg/kg p.o. | 1000 mg/kg p.o. |
| SR 41556 | 0 | 0 | 0 |
| SR 41558 | 0 | 0 | 80 |
| SR 41559 | 0 | 0 | 0 |
| SR 41590 | 0 | 0 | 40 |
| SR 41591 | 0 | 0 | 0 |
| SR 41654 | 0 | 0 | 60 |
| SR 41673 | 0 | 0 | 20 |
| SR 41932 | 0 | 0 | 100 |
| SR 42094 | 0 | 0 | 0 |
| SR 42399 | 0 | 0 | |
| SR 42487 | 0 | 0 | 0 |
| SR 42488 | 0 | 0 | 0 |

Les résultats exprimés en pourcentage d'animaux qui meurent dans les 71 heures consécutives à l'administration orale des produits sont notés dans le tableau précédent.

Les essais ainsi effectués montrent que les produits selon l'invention présentent des propriétés pharmacologiques intéressantes et une faible toxicité. Ils peuvent être utilisés en thérapeutique humaine notamment pour le traitement des affections psychiques, neurologiques, ou neuromusculaires.

En particulier, les produits selon l'invention peuvent être utilisés pour le traitement des troubles de l'humeur ou du comportement: nervosisme, irritabilité ainsi que pour les traitements des états anxieux des insomnies et de l'épilepsie.

Ces produits peuvent être administrés par voie orale ou par voie injectable. Les compositions pharmaceutiques peuvent être solides ou liquides et se présenter par exemple sous forme de comprimés, gélules, granulés, suppositoires ou préparations injectables.

La posologie peut varier dans de larges proportions en particulier suivant le type et la gravité de l'affection à traiter et suivant le mode d'administration. Le plus souvent, chez l'adulte par voie orale, elle est comprise entre 1 mg et 500 mg par jour, éventuellement répartie en plusieurs prises.

A titre d'exemple, on peut indiquer la préparation galénique suivante:

Gélules

| | |
|---|---|
| CM 40907 | 100mg |
| Aérosil | 0,5 mg |
| Stéarate de magnésium | 1,5 mg |
| Amidon STA RX 1500 | 48mg |
| | 150mg |

**Revendications**

pour les états contractants BE, CH, DE, FR, GB, IT, LI, LU, NL, SE.

1. A titre de produits nouveaux, les composés à noyau hétérocyclique diazoté de formule:

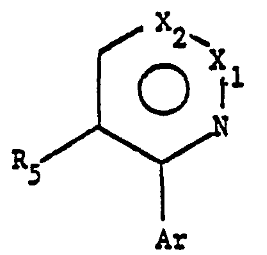

dans laquelle:

- $X_1$ représente N ou le groupe 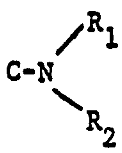 ;

- $X_2$ représente N ou le groupe 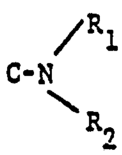

à la condition que $X_1$ et $X_2$ soient différents l'un de l'autre;

- $R_1$ et $R_2$ représentent $-(CH_2)_nOH$; n = 1 à 4

ou

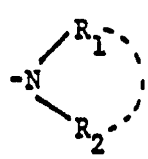

constitue un hétérocycle monoazoté saturé à 5 ou 6 chaînons et portant un substituant en position 3 ou 4 choisi parmi

i) un groupe $-(CH_2)_mOR_6$ où m est égal à 0, 1 ou 2 et $R_6$ représente l'hydrogène ou un groupe 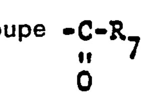 ou

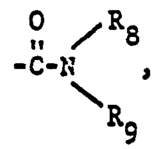

dans lesquels $R_7$ représente un groupe alkyle droit ou ramifié comportant de 1 à 5 atomes de carbone, un groupe cyclopropyle ou un groupe phényle éventuellement substitué par un atome d'halogène, $R_8$ et $R_9$, considérés indépendamment, représentent chacun l'hydrogène ou un groupe alkyle droit ou ramifié comportant de 1 à 5 atomes de carbone;

ii) un groupe oxo (=O);

iii) un groupe spiro dioxolane-1,3 yl-2 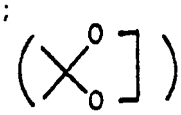 ;

- Ar représente:

. un groupe

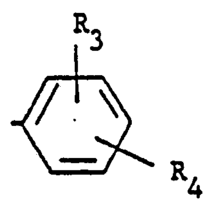

dans lequel

$R_3$ désigne l'hydrogène, un atome d'halogène, un groupe alkyle droit ou ramifié comportant de 1 à 5 atomes de carbone, un groupe alcoxy droit ou ramifié comportant de 1 à 5 atomes de carbone, un groupe trifluorométhyle, un groupe hydroxy, un groupe nitro ou un groupe cyano et

$R_4$ désigne l'hydrogène ou un atome d'halogène;

. un groupe naphtyle non substitué ou monosubstitué par un atome d'halogène;

- $R_5$ désigne l'hydrogène ou un atome de chlore étant entendu que $R_5$ ne peut être un atome de chlore que si $X_2$ représente un atome d'azote;

et les sels desdits composés avec des acides pharmaceutiquement acceptables.

2. Composés selon la revendication 1, caractérisés en ce qu'ils répondent à la formule:

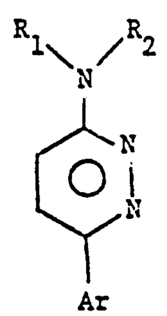

dans laquelle Ar, $R_1$ $R_2$ sont tels que définis dans la revendication 1.

3. Composés selon la revendication 1, caractérisés en ce qu'ils répondent à la formule:

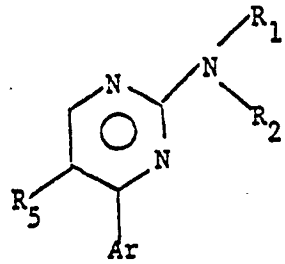

dans laquelle Ar, $R_1$, $R_2$ et $R_5$ sont tels que définis dans la revendication 1.

4. Procédé pour l'obtention des composés selon la revendication 1, caractérisé en ce que l'on utilise comme produit de départ le composé correspondant dans lequel le radical $X_1$ ou $X_2$ qui portera le radical

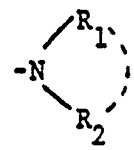

est sous forme d'une cétone, que l'on transforme ladite cétone en le dérivé chloré correspondant, que l'on fait ensuite réagir avec l'amine

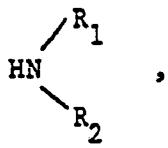

amine dans laquelle $R_1$ et $R_2$ représentent chacun le groupe $-(CH_2)_nOH$, dans lequel n est 1 à 4 ou

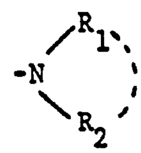

constitue un hétérocycle monoazoté saturé à 5 ou 6 chaînons et portant en position 3 ou 4 un groupe $-(CH_2)_mOH$, dans lequel m est 0, 1 ou 2, ou un groupe spiro dioxolane-1,3-yl-2 et en ce qu'éventuellement:

- le dérivé hydroxylé ainsi obtenu est estérifié par réaction avec un chlorure d'acide ; ou
- le dérivé hydroxylé ainsi obtenu est transformé en carbamate N-monosubstitué, par action d'un isocyanate d'alkyle; ou
- le dérivé hydroxylé ainsi obtenu est transformé en carbamate N,N-disubstitué par action d'un chlorure de carbamoyle; ou

en ce qu'éventuellement on transforme le composé obtenu de formule I dans laquelle le groupe

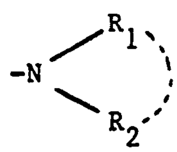

constitue un hétérocycle monoazoté substitué par le groupe spiro dioxolane en le dérivé oxo correspondant, par hydrolyse acide et en ce qu'éventuellement le composé obtenu de formule I dans laquelle $X_2$ représente N et $R_5$ représente H est transformé en celui correspondant de formule I dans laquelle $R_5$ représente un atome de chlore et enfin, en ce qu'éventuellement on salifie les produits obtenus avec les acides organiques ou minéraux.

5. Procédé selon la revendication 4, caractérisé en ce que le produit de départ est une pyridazinone-3, que l'on transforme en la 6-aryl-3-chloro-pyridazine correspondante, laquelle est ensuite substituée par le groupe amine

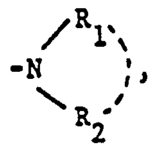

dans lequel $R_1$ et $R_2$ sont tels que définis dans la revendication 1.

6. Procédé selon la revendication 4, caractérisé en ce que le produit de départ est une 6-aryl-pyrimidine-2-one, que l'on transforme en la 2-chloro-6-aryl-pyrimidine correspondante, laquelle est ensuite substituée par le groupe amine

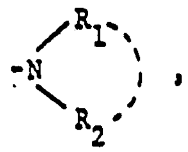

dans lequel $R_1$ et $R_2$ sont tels que définis dans la revendication 1.

7. Médicaments caractérisés en ce qu'ils contiennent en tant que principe actif au moins un composé selon l'une quelconque des revendications 1 à 3.

8. Médicaments actifs sur le système nerveux central, caractérisés en ce qu'ils contiennent en tant que principe actif au moins un composé selon l'une quelconque des revendications 1 à 3.

9. Médicaments selon l'une des revendications 7 ou 8, caractérisés en ce qu'ils sont utilisés par voie orale et comportent unitairement de 1 à 500 mg de produit actif.

10. Médicaments selon l'une des revendications 7 ou 8, caractérisés en ce qu'ils sont conditionnés sous une forme appropriée pour l'administration par voie injectable.

**Revendications**

pour l'état contractant AT

1. Procédé pour la préparation de composés à noyau hétérocyclique diazoté de formule:

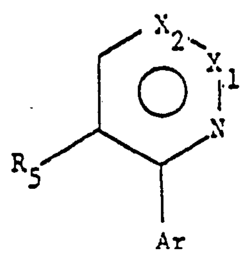

dans laquelle:

- $X_1$ représente N ou le groupe 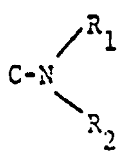 ;

- $X_2$ représente N ou le groupe 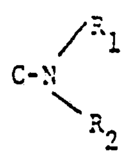

à la condition que $X_l$ et $X_2$ soient différents l'un de l'autre;

- $R_1$ et $R_2$ représentent $-(CH_2)_nOH$; n = 1 à 4 ou

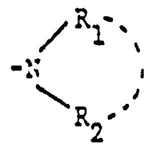

constitue un hétérocycle monoazoté saturé à 5 ou 6 chaînons et portant un substituant en position 3 ou 4 choisi parmi

i) un groupe $-(CH_2)_mOR_6$ où m est égal à 0, 1 ou 2 et

$R_6$ représente l'hydrogène ou un groupe

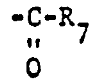

ou

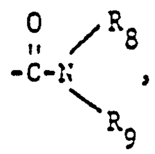

dans lesquels $R_7$ représente un groupe alkyle droit ou ramifié comportant de 1 à 5 atomes de carbone, un groupe cyclopropyle ou un groupe phényle éventuellement substitué par un atome d'halogène, $R_8$ et $R_9$, considérés indépendamment, représentent chacun l'hydrogène ou un groupe alkyle droit ou ramifié comportant de 1 à 5 atomes de carbone;

ii) un groupe oxo (=0);

iii) un groupe spiro dioxolane-1,3 yl-2 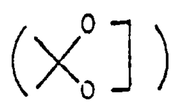 ;

- Ar représente:

un groupe

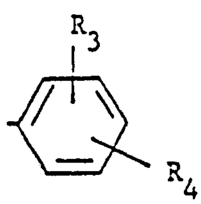

dans lequel

$R_3$ désigne l'hydrogène, un atome d'halogène un groupe alkyle droit ou ramifié comportant de 1 à 5 atomes de carbone, un groupe alcoxy droit ou ramifié comportant de 1 à 5 atomes de carbone, un groupe trifluorométhyle, un troupe hydroxy, un groupe nitro ou un groupe cyano et

$R_4$ désigne l'hydrogène ou un atome d'halogène;

. un groupe naphtyle non substitué ou monosubstitué par un atome d'halogène;

- $R_5$ désigne l'hydrogène ou un atome de chlore, étant entendu que $R_5$ ne peut être un atome de chlore que si $X_2$ représente un atome d'azote;

et les sels desdits composés avec des acides pharmaceutiquement acceptables, caractérisé en ce que l'on utilise comme produit de départ le composé correspondant dans lequel le radical $X_1$ ou $X_2$ qui portera le radical

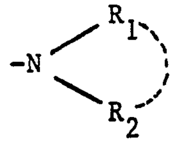

est sous forme d'une cétone, que l'on transforme ladite cétone en le dérivé chloré correspondant, que l'on fait ensuite réagir avec l'amine

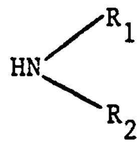,

amine dans laquelle $R_1$ et $R_2$ représentent sentent chacun le groupe $-(CH_2)_nOH$, dans lequel n est 1 à 4 ou

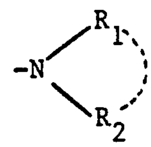

constitue un hétérocycle monoazoté saturé à 5 ou 6 chaînons et pourtant en position 3 ou 4 un groupe $-(CH_2)_mOH$, dans lequel m est 0, 1 ou 2, ou un groupe spiro dioxolane-1,3-yl-2 et en ce qu'éventuellement:

- le dérivé hydroxylé ainsi obtenu est estérifié par réaction avec un chlorure d'acide; ou
- le dérivé hydroxylé ainsi obtenu est transformé en carbamate N-monosubstitué, par action d'un isocyanate d'alkyle; ou
- le dérivé hydroxylé ainsi obtenu est transormé en carbamate N,N-disubstitué par action d'un chlorure de carbamoyle; ou en ce qu'éventuellement on transforme le composé obtenu de formule I dans laquelle le groupe

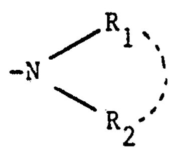

constitue un hétérocycle monoazote substitué par le groupe spiro dioxolane en le dérivé oxo correspondant, par hydrolyse acide et en ce qu'éventuellement le composé obtenu de formule I dans laquelle $X_2$ représente N et $R_5$ représente H est transformé en celui correspondant de formule I dans laquelle $R_5$ représente un atome de chlore et enfin, en ce qu'éventuellement on salifie les produits obtenus avec les acides organiques ou minéraux.

2. Procédé selon la revendication 1, caractérisé en ce que le produit de départ est une pyridazinone-3, que l'on transforme en la 6-aryl-3-chloro-pyridazine correspondante, laquelle est ensuite substituée par le groupe amine

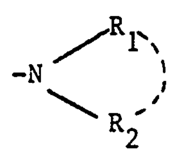,

dans lequel $R_1$ et $R_2$ sont tels que définis dans la revendication 1.

3. Procédé selon la revendication 1, caractérisé en ce que le produit de départ est une 6-aryl-pyrimidine-2-one, que l'on transforme en la 2-chloro-6-aryl-pyrimidine correspondante, laquelle est ensuite substituée par le groupe amine

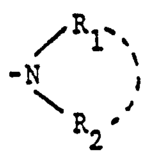,

dans lequel $R_1$ et $R_2$ sont tels que définis dans la revendication 1.

4. Procédé selon la revendication 2 pour la préparation de composés de formule:

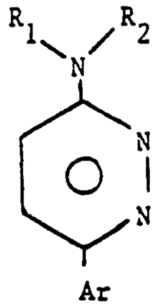

dans laquelle $R_1$, $R_2$ et Ar sont tels que définis dans la revendication 1.

5. Procédé selon la revendication 3, pour la préparation de composés de formule

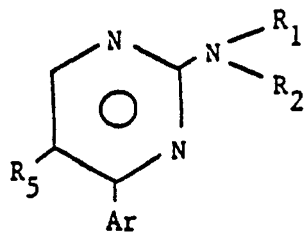

dans laquelle $R_1$, $R_2$, Ar et $R_5$ sont tels que définis dans la revendication 1.

6. Utilisation des composés à noyau hétérocycle diazotés, obtenus selon l'une quelconque des revendications 1 à 5, pour la préparation de médicaments.

7. Utilisation des composés à noyau hétérocycle diazotés, obtenus selon l'une quelconque des revendications 1 à 5, pour la préparation de médicaments actifs sur le système nerveux central.

8. Utilisation des composés à noyau hétérocycle diazotés, obtenus selon l'une quelconque des revendications 1 à 5, pour la préparation de médicaments, utilisés par voie orale et comportant unitairement de 1 à 500 mg de produit actif.

9. Utilisation des composés à noyau hétérocycle diazotés, obtenus selon l'une quelconque des revendicdtions 1 à 5, pour la préparation de médicaments conditionnés sous une forme appropriée pour l'administration par voie injectable.

**Patentansprüche**

für die Vertragsstaaten BE, CH, DE, FR, GB, IT, LI, LU, NL, SE:

1. Als neue Produkte die Verbindungen mit heterozyklischem Kern mit 2 Stickstoffatomen der Formel:.

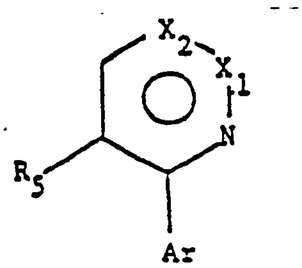

worin:

- $X_1$ für N oder die Gruppe 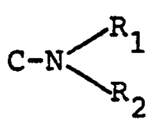

steht;

- $X_2$ für N oder die Gruppe 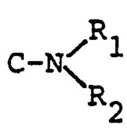

steht, mit der Maßgabe, daß $X_1$ und $X_2$ voneinander unterschiedlich sind;
- $R_1$ und $R_2$ für $-(CH_2)_nOH$ stehen; n = 1 bis 4 oder

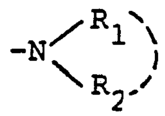

einen Heterozyklus mit einem Stickstoffatom darstellt;
der mit 5 oder 6 Ketten gesättigt ist und in Position 3 oder 4 einen Substituenten trägt, ausgewählt aus
i) einer Gruppe $-(CH_2)_mOR_6$, worin m gleich 0, 1 oder 2 ist und $R_6$ für Wasserstoff oder eine Gruppe

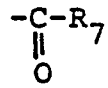

oder

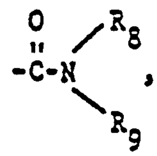

,

steht, worin $R_7$ eine gerade oder verzweigte Alkylgruppe mit 1 bis 5 Kohlenstoffatomen, eine Cyclopropylgruppe oder eine gegebenenfalls durch ein Halogenatom substituierte Phenylgruppe darstellt, $R_8$ und $R_9$, einzeln betrachtet, jeweils Wasserstoff oder eine gerade oder verzweigte Alkylgruppe mit 1 bis 5 Kohlenstoffatomen darstellen;
ii) einer Oxogruppe (=0);
iii) einer Spiro-1,3-Dioxolan-2-yl-gruppe 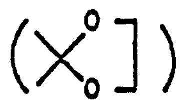 ;

FIG57/20

- Ar bedeutet: eine Gruppe

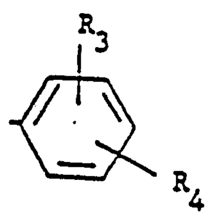

,

worin
$R_3$ für Wasserstoff, ein Halogenatom, eine gerade oder verzweigte Alkylgruppe mit 1 bis 5 Kohlenstoffatomen, eine gerade oder verzweigte Alkoxygruppe mit 1 bis 5 Kohlenstoffatomen, eine Trifluormethylgruppe, eine Hydroxygruppe, eine Nitrogruppe oder eine Cyanogruppe darstellt, und
$R_4$ Wasserstoff oder ein Halogenatom bedeutet;
. eine nicht substituierte oder durch ein Halogenatom monosubstituierte Naphthylgruppe;
- $R_5$ für Wasserstoff oder ein Chloratom steht, mit der Maßgabe, daß $R_5$ nur ein Chloratom sein kann, wenn $X_2$ ein Stickstoffatom darstellt;
und die Salze dieser Verbindungen mit pharmazeutisch akzeptablen Säuren.
2. Verbindungen nach Anspruch 1, dadurch gekennzeichnet, daß sie der Formel:

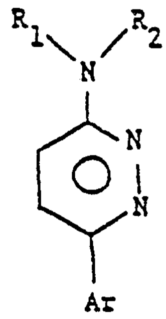

entsprechen, worin Ar, $R_1$, $R_2$ wie in Anspruch 1 definiert sind.

3. Verbindungen nach Anspruch 1, dadurch gekennzeichnet, daß sie der Formel:

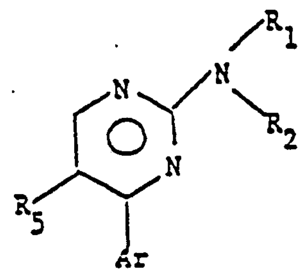

entsprechen, worin Ar, $R_1$, $R_2$ und $R_5$ wie in Anpruch 1 definiert sind.

4. Verfahren zur Herstellung der Verbindungen nach Anspruch 1, dadurch gekennzeichnet, daß man als Ausgangsprodukt die entsprechende Verbindung einsetzt, worin das Radikal $X_1$ oder $X_2$, das die Gruppe

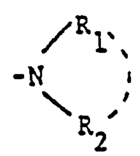

tragen soll, in Form eines Ketons vorliegt, daß man das Keton in das entsprechende chlorierte Derivat überführt, daß man es danach mit dem Amin

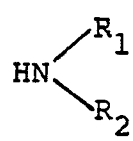

reagieren läßt, in welchem Amin $R_1$ und $R_2$ jeweils für die Gruppe $-(CH_2)_nOH$ stehen, worin n 1 bis 4 ist oder

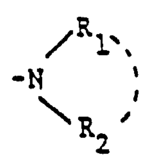

einen mit 5 oder 6 Ketten gesättigten Heterozyklus mit 1 Stickstoffatom darstellt und in Position 3 oder 4 eine Gruppe $-(CH_2)_mOH$, worin m 0,1 oder 2 ist, oder eine Spiro-1,3-dioxolan-2-yl-gruppe trägt, und daß gegebenenfalls:

- das so erhaltene hydroxylierte Derivat durch Umsetzung mit einem Säurechlorid verestert wird; oder
- das so erhaltene hydroxylierte Derivat durch Einwirken eines Alkylisocyanats in ein N-monosubstituiertes Carbamat übergeführt wird; oder
- das so erhaltene hydroxylierte Derivat durch Einwirken eines Carbamoylchlorids in das N,N-disubstituierte Carbamat übergeführt wird; oder

daß man gegebenenfalls die erhaltene Verbindung der Formel I, worin die Gruppe

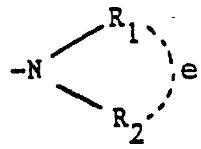 einen dui

einen durch die Spiro-dioxolangruppe substituierten Heterozyklus mit 1 Stickstoffatom darstellt, durch saure Hydrolyse in das entsprechende Oxoderivat überführt, und daß gegebenenfalls die erhaltene Verbindung der Formel I, worin $X_2$ für N und $R_5$ für H stehen, in jene der Formel I übergeführt wird, worin $R_5$ ein Chloratom darstellt, und daß man schließlich die erhaltenen Produkte mit organischen oder Mineralsäuren in Salze umwandelt.

5. Verfahren nach Anspruch 4, dadurch gekennzeichnet, daß das Ausgangsprodukt ein 3-Pyridazinon ist, das man in das entsprechende 6-Aryl-3-chlorpyridazin überführt, welches danach durch die Aminogruppe

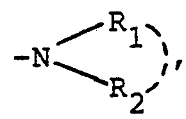

worin $R_1$ und $R_2$ wie in Anspruch 1 definiert sind, substituiert wird.

6. Verfahren nach Anspruch 4, dadurch gekennzeichnet, daß das Ausgangsprodukt 6-Aryl-pyrimidin-2-on ist, das man in das entsprechende 2-Chlor-6-aryl-pyrimidin überführt, welches danach durch die Aminogruppe

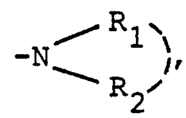

worin $R_1$ und $R_2$ wie in Anspruch 1 definiert sind, substituiert wird.

7. Medikamente, dadurch gekennzeichnet, daß sie als Wirkstoff mindestens eine Verbindung nach einem der Ansprüche 1 bis 3 enthalten.

8. Medikamente, die auf das Zentralnervensystem aktiv sind, dadurch gekennzeichnet, daß sie als Wirkstoff mindestens eine Verbindung nach einem der Ansprüche 1 bis 3 enthalten.

9. Medikamente nach einem der Ansprüche 7 oder 8, dadurch gekennzeichnet, daß sie auf oralem Weg verwendet werden und als Einheit 1 bis 500 mg aktives Produkt umfassen.

10. Medikamente nach einem der Ansprüche 7 oder 8, dadurch gekennzeichnet, daß sie in für Verabreichung auf Injektionsweg geeigneter Form konditioniert sind.

**Patentansprüche**

für den Vertragsstaat AT:

1. Verfahren zur Herstellung von Verbindungen mit heterozyklischem Kern mit 2 Stickstoffatomen der Formel:

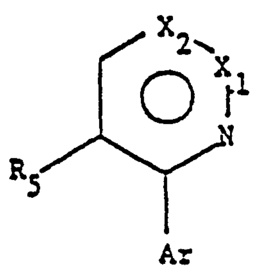

worin:

- $X_1$ für N oder die Gruppe 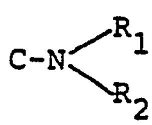

steht;

- $X_2$ für N oder die Gruppe 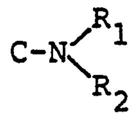

FIG69/20

steht, mit der Maßgabe, daß $X_1$ und $X_2$ voneinander unterschiedlich sind;
- $R_1$ und $R_2$ für $-(CH_2)_nOH$ stehen; n = 1 bis 4 oder

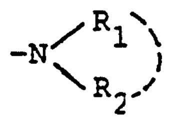

einen Heterozyklus mit einem Stickstoffatom darstellt;
der mit 5 oder 6 Ketten gesättigt ist und in Position 3 oder 4 einen Substituenten trägt, ausgewählt aus
i) einer Gruppe $-(CH_2)_mOR_6$, worin m gleich 0, 1 oder 2 ist und $R_6$ für Wasserstoff oder eine Gruppe

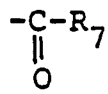

oder

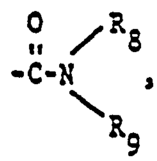 ,

steht, worin $R_7$ eine gerade oder verzweigte Alkylgruppe mit 1 bis 5 Kohlenstoffatomen, eine Cyclopropylgruppe oder eine gegebenenfalls durch ein Halogenatom substituierte Phenylgruppe darstellt, $R_8$ und $R_9$, einzeln betrachtet, jeweils Wasserstoff oder eine gerade oder verzweigte Alkylgruppe mit 1 bis 5 Kohlenstoffatomen darstellen;
ii) einer Oxogruppe (=0);
iii) einer Spiro-1,3-Dioxolan-2-yl-gruppe 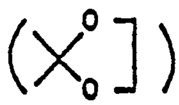 ;

- Ar bedeutet:
. eine Gruppe,

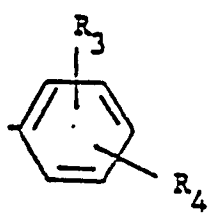 ,

worin
$R_3$ für Wasserstoff, ein Halogenatom, eine gerade oder verzweigte Alkylgruppe mit 1 bis 5 Kohlenstoffatomen, eine gerade oder verzweigte Alkoxygruppe mit 1 bis 5 Kohlenstoffatomen, eine Trifluormethylgruppe, eine Hydroxygruppe, eine Nitrogruppe oder eine Cyanogruppe darstellt, und
$R_4$ Wasserstoff oder ein Halogenatom bedeutet;

. eine nicht substituierte oder durch ein Halogenatom monosubstituierte Naphthylgruppe;

- $R_5$ für Wasserstoff oder ein Chloratom steht, mit der Maßgabe, daß $R_5$ nur ein Chloratom sein kann, wenn $X_2$ ein Stickstoffatom darstellt;

und der Salze dieser Verbindungen mit pharmazeutisch akzeptablen Säuren, dadurch gekennzeichnet, daß man als Ausgangsprodukt die entsprechende Verbindung einsetzt, worin das Radikal $X_1$ oder $X_2$, das die Gruppe

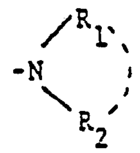

tragen soll, in Form eines Ketons vorliegt, daß man das Keton in das entsprechende chlorierte Derivat überführt, daß man es danach mit dem Amin

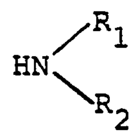

reagieren läßt, in welchem Amin $R_1$ und $R_2$ jeweils für die Gruppe $-(CH_2)_nOH$ stehen, worin n 1 bis 4 ist oder

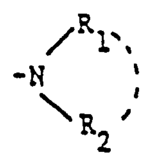

einen mit 5 oder 6 Ketten gesättigten Heterozyklus mit 1 Stickstoffatom darstellt und in Position 3 oder 4 eine Gruppe $-(CH_2)_mOH$, worin m 0,1 oder 2 ist, oder eine Spiro-1,3-dioxolan-2-yl-gruppe trägt, und daß gegebenenfalls:

- das so erhaltene hydroxylierte Derivat durch Umsetzung mit einem Säurechlorid verestert wird; oder
- das so erhaltene hydroxylierte Derivat durch Einwirken eines Alkylisocyanats in ein N-monosubstituiertes Carbamat übergeführt wird; oder
- das so erhaltene hydroxylierte Derivat durch Einwirken eines Carbamoylchlorids in das N,N-disubstituierte Carbamat übergeführt wird; oder daß man gegebenenfalls die erhaltene Verbindung der Formel I, worin die Gruppe

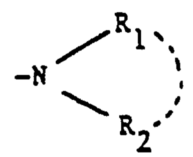

einen durch die Spiro-dioxolangruppe substituierten Heterozyklus mit 1 Stickstoffatom darstellt, durch saure Hydrolyse in das entsprechende Oxoderivat überführt, und daß gegebenenfalls die erhaltene Verbindung der Formel I, worin $X_2$ für N und $R_5$ für H stehen, in jene der Formel I übergeführt wird, worin $R_5$ ein Chloratom darstellt, und daß man schließlich die erhaltenen Produkte mit organischen oder Mineralsäuren in Salze umwandelt.

2. Verfahren nach Anspruch 1, dadurch gekennzeichnet, daß das Ausgangsprodukt ein 3-Pyridazinon ist, das man in das entsprechende 6-Aryl-3-chlorpyridazin überführt, welches danach durch die Aminogruppe

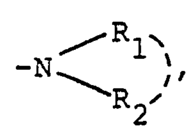

worin $R_1$ und $R_2$ wie in Anspruch 1 definiert sind, substituiert wird.

3. Verfahren nach Anspruch 1, dadurch gekennzeichnet, daß das Ausgangsprodukt 6-Aryl-pyrimidin-2-on ist, das man in das entsprechende 2-Chlor-6-aryl-pyrimidin überführt, welches danach durch die Aminogruppe

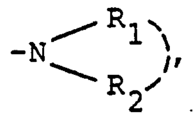

worin $R_1$ und $R_2$ wie in Anspruch 1 definiert sind, substituiert wird.

4. Verfahren nach Anspruch 2 zur Herstellung von Verbindungen der Formel:

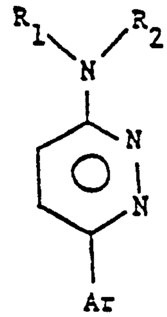

worin $R_1$, $R_2$ und Ar wie in Anspruch 1 definiert sind.

5. Verfahren nach Anspruch 3 zur Herstellung von Verbindungen der Formel

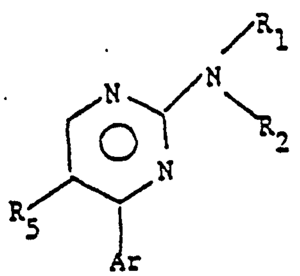

worin $R_1$, $R_2$, Ar und $R_5$ wie in Anspruch 1 definiert sind.

6. Verwendung der nach einem der Ansprüche 1 bis 5 erhaltenen Verbindungen mit heterozyklischem Kern mit 2 Stickstoffatomen zur Herstellung von Medikamenten.

7. Verwendung der nach einem der Ansprüche 1 bis 5 erhaltenen Verbindungen mit heterozyklischem Kern mit 2 Stickstoffatomen zur Herstellung von Medikamenten, die auf das Zentralnervensystem aktiv sind.

8. Verwendung der nach einem der Ansprüche 1 bis 5 erhaltenen Verbindungen mit heterozyklischem Kern mit 2 Stickstoffatomen zur Herstellung von Medikamenten, die auf oralem Weg verwendet werden und als Einheit 1 bis 500 mg aktives Produkt umfassen.

9. Verwendung der nach einem der Ansprüche 1 bis 5 erhaltenen Verbindungen mit heterozyklischem Kern mit 2 Stickstoffatomen zur Herstellung von Medikamenten, die in einer für Verabreichung auf Injektionsweg geeigneten Form konditioniert sind.

**Claims**

for the contracting states: BE, CH, DE, FR, GB, IT, LI, LU, NL, SE

1. By way of novel products, the compounds having a heterocyclic necleus containing two nitrogen atoms, of formula:

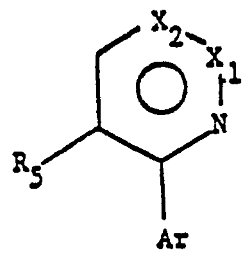

in which:
- $X_1$ represents N or the gorup 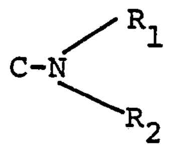 ;

- $X_2$ represents N or the group 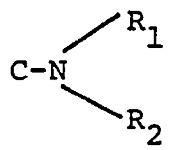

on condition that $X_1$ and $X_2$ are different from each other;
- $R_1$ and $R_2$ represent $-(CH_2)_nOH$; $n = 1$ to 4 or

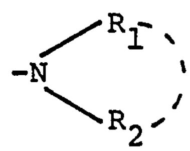

constitutes a saturated heterocyclic with 5 or 6 groupings containing a nitrogen atom and bearing a substituent in 3 or 4 position selected from:
i) a $-(CH_2)_mOR_6$ group where m is equal to 0,1 or 2 and $R_6$ represents hydrogen or a group

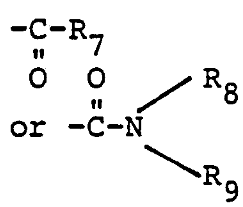
or

in which $R_7$ represents a straight or branched alkyl group having from 1 to 5 carbon atoms, a cyclopropyl group or a phenyl group possibly substituted by an atom of halogen, and $R_8$ and $R_9$ considered independently each represent hydrogen or a straight or branched alkyl group having from 1 to 5 carbon atoms,
ii) an oxo (=0) group;
iii) a spiro -1,3-dioxolane-2-yl 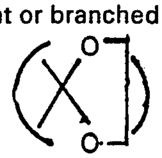

group;
- Ar represents:
. a group

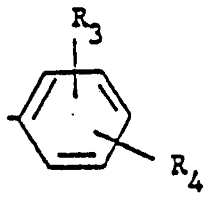

in which

$R_3$ destignates hydrogen, an atom of halogen, a straight or branched alkyl group, having from 1 to 5 atoms of carbon a straight or branched alkoxy group, having from 1 to 5 atoms of carbon, a trifluoromethyl group, a hydroxy group, a nitro group or a cyano group and $R_4$ destignates hydrogen or an atom of halogen;

. a naphthyl group non-substituted or monosubstituted by an atom of halogen;

$R_5$ designates hydrogen or an atom of chlorine, it being understood that $R_5$ can be an atom of chlorine only if $X_2$ represents an atom of nitrogen, and the salts of said compounds with pharmaceutically acceptable acids.

2. The compounds according to claim 1, characterized in that they correspond to formula:

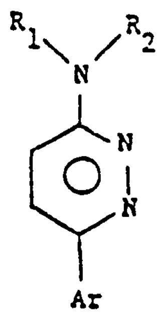

in which Ar, $R_1$, $R_2$ are as defined in claim 1.

3. The compounds according to claim 1, characterized in that they correspond to formula:

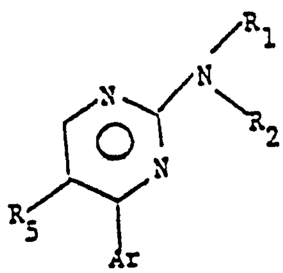

in which Ar, $R_1$, $R_2$ and $R_5$ are as defined in claim 1.

4. A process for obtaining the compounds according to claim 1, characterized in that the starting product is constituted by the corresponding compound in which the radical $X_1$ or $X_2$ which will bear the radical

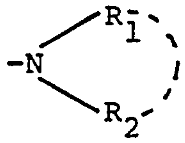

is in the form of a ketone, said ketone is converted into the corresponding chlorinated derivative, which is then reacted with the amine

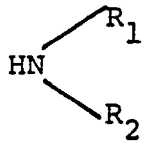,

in which amine $R_1$ and $R_2$ each represent the group-$(CH_2)_nOH$ in which n is 1 to 4 or

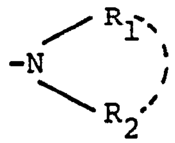

constitutes a saturated heterocycle with 5 or 6 groupings containing a nitrogen atom and bearing in 3 or 4 position a $-(CH_2)_mOH$ group in which m is 0, 1 or 2, or a spiro -1,3-dioxolane-2-yl group and in that optionally

- the hydroxylated derivative thus obtained is esterified by reaction with an acid chloride; or
- the hydroxylated terivative thus obtained is converted into N-monosubstituted carbamate by action of an alkyl isocyanate; or - the hydroxylated derivative thus obtained is converted into N,N-disubstituted carbamate by action of a carbamoyl chloride: or in that the obtained compound of formula (I) in which the

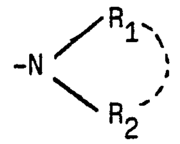

group constitutes a heterocycle containing a nitrogen atom substituted by the spiro dioxolane group is optionally converted into the corresponding oxo derivative, by acid hydrolysis and in that optionally the resulting compound of formula I in which $X_2$ represents N and $R_5$ represents H is converted into the corresponding product of formula I in which $R_5$ represents an atom of chlorine and finally, in that the products thus obtained are possibly salified with the organic or mineral acids.

5. The process according to claim 4, characterized in that the starting product is a 3-pyridazinone which is converted into the corresponding 6-aryl-3-chloro-pyridazine, which is then substituted by the amine

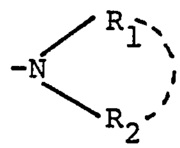

group in which $R_1$ and $R_2$ are as defined in claim 1.

6. The Process according to claim 4, characterized in that the starting product is a 6-aryl-pyrimidine-2-one, which is converted into the corresponding 2-chloro-6-aryl-pyrimidine, which is then substituted by the amine

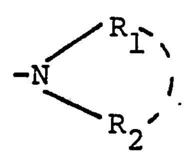

group in which $R_1$ and $R_2$ are as defined in claim 1.

7. Drugs characterized in that they contain as active principle at least one compound according to any one of claims 1 to 3.

8. Drugs active on the central nervous system, characterized in that they contain as active principle at least one compound according to any one of claims 1 to 3.

9. The drugs according to any one of claims 7 or 8 characterized in that they are administered by the oral route and comprise per unit dose from 1 to 500 mg of active product.

10. The drugs according to any one of claims 7 or 8, characterized in that they are in a form appropriate for administration by the injectable route.

**Claims**

for the contracting state: AT

1. Process for preparing compounds having a heterocyclic nucleus containing two nitrogen atoms, of formula:

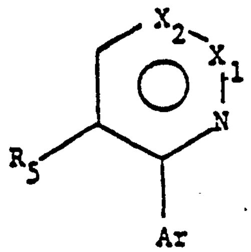

in which:

- $X_1$ represents N or the group 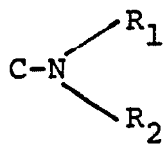 ;

- $X_2$ represents N or the group 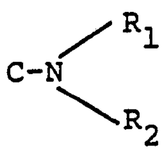

on condition that $X_1$ and $X_2$ are different from each other;

- $R_1$ and $R_2$ represent $-(CH_2)_nOH$; n = 1 to 4 or

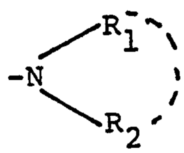

constitutes a saturated heterocycle with 5 or 6 groupings containing a nitrogen atom and bearing a substituent in 3 or 4 position selected from:

i) a $-(CH_2)_mOR_6$ group where m is equal to 0, 1 or 2 and $R_6$ represents hydrogen or a group 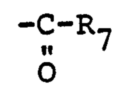

or 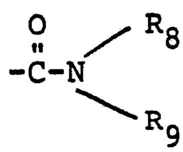

in which $R_7$ represents a straight or branched alkyl group, having from 1 to 5 atoms of carbon, a cyclopropyl group or a phenyl group possibly substituted by an atom of halogen, $R_8$ and $R_9$ considered independently each represent hydrogen or a straight or branched alkyl group having from 1 to 5 atoms of carbon;

ii) an oxo (=0) group;

iii) a spiro -1,3-dioxolane-2-y 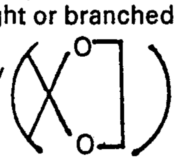

group;
- Ar represents:
. a group

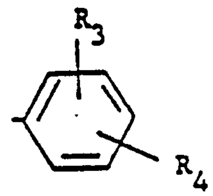

in which
$R_3$ designates hydrogen, an atom of halogen, a straight or branched alkyl group having from 1 to 5 atoms of carbon, a straight or branched alkoxy group having from 1 to 5 atoms of carbon, a trifluoromethyl group, a hydroxy group, a nitro group or a cyano group and $R_4$ designates hydrogen or an atom of halogen;
. a naphthyl group non-substituted or monosubstituted by an atom of halogen;
- $R_5$ designates hydrogen or an atom of chlorine, it being understood that $R_5$ can be an atom of chlorine only if $X_2$ represents an atom of nitrogen; and the salts of said compounds with pharmaceutically acceptable acids, characterized in that the starting product used is constituted by the corresponding compound in which the radical $X_1$ or $X_2$ which will bear the radical

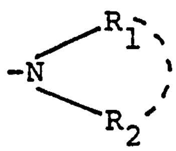 is

in the form of a ketone, said ketone is converted into the corresponding chlorinated derivative, which is then reacted with the amine

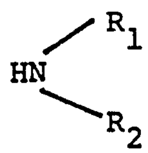 ,

in which amine $R_1$ and $R_2$ each represent the group $-(CH_2)_nOH$ in which n is 1 to 4 or

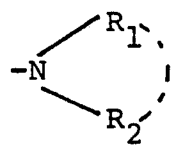

constitutes a saturated heterocycle with 5 or 6 groupings containing a nitrogen atom and bearing in 3 or 4 position a $-(CH_2)_mOH$ group in which m is 0, 1 or 2 or a spiro -1,3-dioxolane-2-yl group and in that optionally,
- the hydroxylated derivative thus obtained is esterified by reaction with an acid chloride; or
- the hydroxylated derivative thus obtained is converted into N-monosubstituted carbamate, by action of an alkyl isocyanate; or
- the hydroxylated derivative thus obtained is converted into N,N-disubstituted carbamate by action of a carbamoyl chloride; or in that the resulting compound of formula I in which the

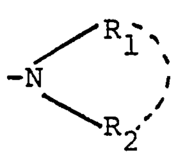

group constitutes a heterocycle containing a nitrogen atom substituted by the spiro dioxolane group is optionally converted into the corresponding oxo derivative, by acid hydrolysis and in that optionally the resulting compound of formula I in which $X_2$ represents N and $R_5$ represents H is converted into the corresponding product of formula I in which $R_5$ represents an atom of chlorine and finally, in that the products thus obtained are salified with the organic or mineral acids.

2. The process according to claim 1, characterized in that the starting product is a 3-pyridazinone which is converted into the corresponding 6-aryl-3-chloro-pyridazine, which is then substituted by the amine

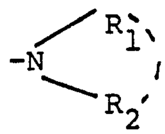

group in which $R_1$ and $R_2$ are as defined in claim 1.

3. The process according to claim 1 characterized in that the starting product is a 6-aryl-pyrimidine-2-one, which is converted into the corresponding 2-chloro-6-aryl pyrimidine, which is then substituted by the amine

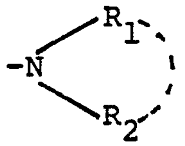

group in which $R_1$ and $R_2$ are as defined in claim 1.

4. Process according to claim 2, for the preparation of compounds of formula

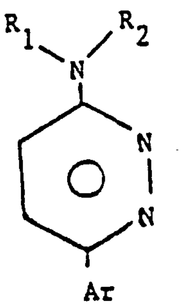

in which $R_1$, $R_2$ and Ar are as defined in claim 1.

5. Process according to claim 3, for the preparation of compounds of formula:

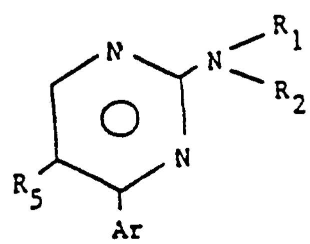

in which Ar, $R_1$, $R_2$ and $R_5$ are as defined in claim 1.

6. Use of the compounds, having a heterocyclic nucleus containing two nitrogen atoms, obtained according to any one of claims 1 to 5, for the preparation of drugs.

7. Use of the compounds, having a heterocyclic nucleus, containing two nitrogen atoms, obtained according to any one of claims 1 to 5, for the preparation of drugs active on the central nervous system.

8. Use of the compounds having a heterocyclic nucleus, containing two nitrogen atoms, obtained according to any one of claims 1 to 5, for the preparation of druge, administered by the oral route and comprising per unit dose from 1 to 500 mg of active product.

9. Use of the compounds having a heterocyclic nucleus, containing two nitrogen atoms, obtained according to any one of claims 1 to 5, for the preparation of drugs packed in a form appropriate for administration by the injectable route.